(19) Europäisches Patentamt
European Patent Office
Office européen des brevets

(11) EP 3 858 330 A1

(12) # EUROPEAN PATENT APPLICATION

(43) Date of publication:
**04.08.2021 Bulletin 2021/31**

(21) Application number: **20154946.6**

(22) Date of filing: **31.01.2020**

(51) Int Cl.:
*A61K 9/107* (2006.01) *A61K 9/19* (2006.01)
*A61K 47/34* (2017.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Freie Universität Berlin**
**14195 Berlin (DE)**

(72) Inventors:
- **HAAG, Rainer**
  **12209 Berlin (DE)**
- **FERRARO, Magda**
  **12163 Berlin (DE)**
- **MOHAMMADIFAR, Ehsan**
  **10407 Berlin (DE)**
- **SCHIRNER, Michael**
  **13158 Berlin (DE)**

(74) Representative: **Maikowski & Ninnemann Patentanwälte Partnerschaft mbB**
**Postfach 15 09 20**
**10671 Berlin (DE)**

(54) **HYPERBRANCHED POLYESTER POLYOL DERIVATIVE AS DRUG SOLUBILIZER**

(57) The present invention relates to a composition comprising a hyperbranched polyester polyol derivative and a heterocyclic tumor signal transduction inhibitor comprising at least one cyclic amine. The hyperbranched polyester polyol derivative is obtainable by a method comprising the following steps: a) reacting only glycidol and ε-caprolactone at a temperature lying in a range of between 40 °C and 140 °C to obtain a hyperbranched polyester polyol derivative in which caprolactone residues and glycerol residues are randomly arranged; b) reacting the hyperbranched polyester polyol derivative of step a) with a sulfation reagent to obtain a sulfated hyperbranched polyester polyol as hyperbranched polyester polyol derivative.

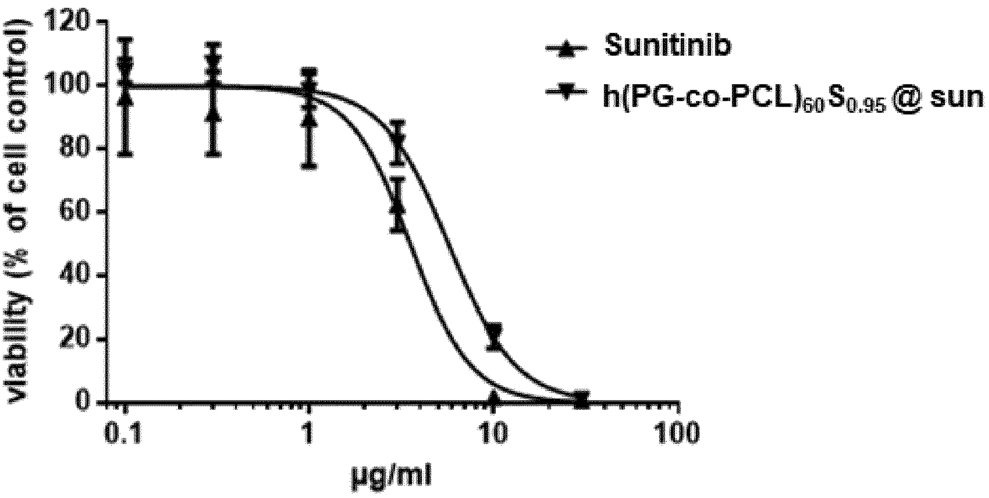


FIG 1

EP 3 858 330 A1

## Description

The present invention relates to a composition comprising a hyperbranched polyester polyol derivative and a heterocyclic tumor signal transduction inhibitor of a specific group according to claim 1, to medical uses of such a composition according to claims 6 and 7 and to a method for manufacturing such a composition according to the preamble of claim 8.

Therapeutic effectiveness of drug treatment is directly related to the bioavailability of drugs at the receptor site. With respect to the treatment of cancer, receptors for most drugs are present on the tumor cell membrane or in the cytosol within the cells. High drug concentration at the receptor site can usually be achieved by macromolecular drugs such as polypeptides and proteins which carry a targeting unit. Here, circulation half-life and binding strength determine the degree of drug accumulation. Unfortunately, most of the drug targets of interest in chronic diseases such as cancer and inflammation cannot be occupied by macromolecular drugs as they are expressed in the intracellular compartment which is not easily accessible for macromolecular drugs.

Intracellular drug targets are usually occupied by small synthetic substances with a medium molecular weight of 550 Da in a range of 100 to 1000 Da. In the medical routine, these drugs are applied once or twice daily as tablet or capsule. Physicochemical properties of the drug molecule such as the degree of hydrophobicity and protonation of small molecules determine the uptake from the gastrointestinal tract (oral bioavailability) and distribution within the body. However, once a drug substance is circulating in the blood stream, fast elimination from the blood and wide distribution outside the blood volume is not desired.

Most drugs which occupy intracellular receptors are hydrophobic, meaning that they have greater solubility in octanol than in water. One of the major hurdles in the pharmaceutical development of novel tumor signaling inhibitors is the hydrophobicity and increasing complexity of the chemical drug substances. The percentage of newly discovered chemical lead structures which cannot be developed as oral drug form is continuously increasing. In this regard, solubilization of the drug molecules is indispensable as only the solubilized chemical structure can be resorbed from the gastrointestinal tract.

Furthermore, hydrophobic drugs show an unfavorable distribution in the body. They leave the blood circulation and often enrich in the extravascular space, do not achieve high maximal drug concentrations in the blood and do not significantly accumulate in the diseased tissue. At the very end, only 1 out of 50.000 drug molecules reaches the primary tumor at the time point of clinical diagnosis. This disadvantageous distribution behavior of hydrophobic drugs minimizes the therapeutic window and limits the outcome of treatment.

Up to 60% of new chemical entities (NCEs) discovered by the pharmaceutical industry today are hydrophobic compounds. The solubility issues complicating the delivery of these new drugs also affect the delivery of many existing drugs. Relative to highly soluble compounds, low drug solubility often manifests itself in a host of in vivo consequences including decreased bioavailability, increased chance of food effect, incomplete release from the dosage form, and higher interpatient variability. Poorly soluble compounds also present many in vitro formulation obstacles, such as severely limited choices of delivery technologies and increasingly complex dissolution testing with limited or poor correlation to the in vivo absorption. These in vivo/in vitro correlations are often sufficiently formidable to halt the development of many newly synthesized compounds due to solubility issues.

Poorly soluble drugs such as nifedipine and felodipine have motivated the development of drug delivery technologies to overcome the obstacles to their solubilization through either chemical or mechanical modification of the environment surrounding the drug molecule or physically altering the macromolecular characteristics of aggregated drug particles. These technologies include both traditional methods of solubility enhancement such as particle size reduction via comminution and spray drying, micellar solubilization, and cyclodextrin mediated inclusion complexes.

Another drawback of the hydrophobicity of most small drug molecules is the poor water solubility. Solubilization of hydrophobic drugs is one of the most challenging issues in pharmaceutical development. A certain level of aqueous solubility is required for adequate absorption in the gastrointestinal tract or for manufacturing of certain drug forms or drug combinations. Non-active pharmaceutical ingredients, such as Tween® 80 and Cremophor® have been widely used in formulations to increase drug solubility. These surfactants solubilize hydrophobic drugs by forming micellar structures in aqueous media. Unfortunately, these surfactants may trigger allergic reactions when administered to patients. Novel PEG-based polymers have been established to improve safety of drug formulation of poorly water-soluble drugs. However, PEG-based micellar drug formulations disintegrate in the blood circulation when the concentration is below their critical micelle concentration (CMC), resulting in a rapid release of the drug.

Despite a wide range of established pharmaceutical methods for solubilizing hydrophobic drugs, there remains a need for new non-active pharmaceutical ingredients and improved pharmaceutical formulation methods.

First, techniques in the art often use harmful substances within the formulation process. Therefore, purification of the product is often difficult and expensive. Methods for drug solubilization in aqueous media without using harmful solvents would be especially needed.

Second, pharmaceutical formulations for the increasing number poorly water-soluble drugs to enable oral

dosage forms are needed. Especially, the oral bioavailability of poorly water-soluble drugs with a molecular weight greater than 400 Da strongly depends on the sufficient solubility of the drug substance. Furthermore, solubility of the drug substance is also a strong need for parenteral application if the bioavailability after oral dosage is to low or high blood concentrations of the drug substance are needed.

Third, formulation systems are needed to formulate several drugs with different physicochemical properties simultaneously which would hinder each other within one formulation. This is also extremely important for the fixed combination of different drug molecules which usually have a highly variable pharmacokinetic behavior in the organism. A delivery system for fixed combination of two or more hydrophobic drug substances may enable controlled uptake, distribution and drug release in the organism with significantly lower drug level variability.

Dendrimers represent a novel type of polymeric material that has generated much interest in pharmaceutical industry due to their unique structure and properties. Dendrimer-mediated solubility enhancement was shown for a great number of hydrophobic molecules. The degree of solubilization mainly depends on factors such as molecular size, dendrimer concentration, pH, temperature, and terminal functionality. However, available dendrimers do not provide an efficient solubilization meaning a significant access of polymer is needed to solubilize a certain amount of hydrophobic drug substance. This low loading efficacy is a major hurdle for application of drug-loaded dendrimers to patients as toxicity from the polymer must be expected.

Thus, there is still a strong need in the art for a method for achieving aqueous solubility to a hydrophobic drug such that the drug may be administered in a therapeutically effective manner.

WO 2019/096782 A1 describes hyperbranched polyester polyol derivatives and their combined application as carrier for a pharmaceutically active substance and as a pharmaceutically active substance themselves.

It is an object of the present invention to provide novel uses of the hyperbranched polyester polyol derivatives described in WO 2019/096782 A1.

It was of surprisingly found by the present inventors that the hyperbranched polyester polyol derivatives described in WO 2019/096782 A1 are particularly appropriate for encapsulating a specific group of pharmaceutically active compounds, namely, of heterocyclic tumor signal transduction inhibitors comprising at least one cyclic amine.

Before going into details, certain terms used throughout this disclosure will be defined.

The terms "primary amine", "secondary amine", and "tertiary amine" are used herein to refer to compounds and functional groups that contain a basic nitrogen atom with a lone electron pair. Amines are formally derivatives of ammonia, wherein one or more hydrogen atoms have been replaced by a chemical substituent. Amines can be classified according to the nature and number of substituents on nitrogen. Aliphatic amines contain only H and alkyl substituents. Aromatic amines have the nitrogen atom connected to an aromatic ring or incorporated into an aromatic ring (system). Cyclic amines comprise a nitrogen incorporated into an aromatic or aliphatic ring (system), i.e., incorporated into a heterocycle.

Amines are generally organized into three subcategories based on the number of carbon atoms adjacent to the nitrogen:

- Primary (1°) amines arise when one of three hydrogen atoms in ammonia is replaced by an alkyl or aromatic group.
- Secondary (2°) amines have two organic substituents bound to the nitrogen together with one hydrogen.
- Tertiary (3°) amines. In tertiary amines, nitrogen has three organic substituents.
- Cyclic amines represent a fourth subcategory determined by the connectivity of the substituents attached to the nitrogen. Cyclic amines are either secondary or tertiary amines.

The term "drug", "biologically active molecule", "biologically active moiety" or "biologically active agent", when used herein, means any substance which can affect any physical or biochemical properties of a biological organism, including but not limited to viruses, bacteria, fungi, plants, animals, and humans. As used herein, biologically active molecules include any substance intended for diagnosis, cure mitigation, treatment, or prevention of disease in humans or other animals, or to otherwise enhance physical or mental well-being of humans or animals.

The term "tumor signal transduction inhibitor" relates to a substance that is able to inhibit signal transduction from the tumor cell membrane into the cytosol and/or the nucleus of tumor cells, or to inhibit tumor cell signaling between tumor cells (and potentially also between tumor cells and other cells or also between cells other than tumor cells). Tumor signal transduction inhibitors belong to the group of anti-tumor agents, i.e., to a subclass of drugs or biologically active molecules.

"Hydrophobic" refers to molecules having a greater solubility in octanol than in water. Conversely, "hydrophilic" refers to molecules having a greater solubility in water than in octanol.

The object underlying the present invention is solved by a composition comprising on the one hand a specific

hyperbranched polyester polyol derivative and on the other hand a heterocyclic tumor signal transduction inhibitor comprising at least one cyclic amine. The hyperbranched polyester polyol derivative is obtainable by a method comprising the steps explained in the following.

In a first step, only glycidol and ε-caprolactone are reacted at a temperature lying in a range of between 40 °C and 140 °C to obtain a hyperbranched polyester polyol derivative in which caprolactone residues and glycerol residues are randomly arranged.

In a second step, the hyperbranched polyester polyol derivative of the first step is reacted with a sulfation reagent to obtain a sulfated hyperbranched polyester polyol as hyperbranched polyester polyol derivative.

It was surprisingly found that certain hydrophobic molecules comprising a cyclic amine can be solubilized and delivered without a pharmaceutical formulation based on nanoparticle, multimolecular micelles or liposomes. Instead, the sulfated, hyperbranched polyester polyol derivatives are suited for solubilization of such hydrophobic molecules having at least one cyclic amine function while molecules without cyclic amine functions cannot be well solubilized.

Thus, the inventors of the present invention have surprisingly found a methodology of solubilizing and delivering hydrophobic, poorly water-soluble molecules having at least one cyclic amine.

Surprisingly, it was found that specific hyperbranched polyester polyol derivatives carrying a plurality of sulfate groups on the surface can directly solubilize strong hydrophobic drug molecules under aqueous conditions without the use of organic solvents provided that the drug molecules have one or more cyclic amine functions. Furthermore, very surprisingly, the sulfated, hyperbranched polyester polyol derivative may bind and/or entrap suited hydrophobic drugs to the polymer to enable delivery to a patient.

With respect to an application of aspects of the invention, the use of sulfated polyglycerol derivatives for organic solvent-free solubilization of hydrophobic drugs is not limited to a certain form of drug formulation. The claimed composition can be applied for the development and manufacturing of an oral dosage drug form and for the development of a parental dosage drug form and any other form applicable for nasal, buccal, pulmonary, ocular, rectal, cutaneous, subcutaneous, intramuscular or intrathecal application.

The hyperbranched polyester polyol derivatives (dendritic polyol derivatives) to be used according to the presently described and claimed invention are spherical macromolecules comprising an interior space and a surface carrying sulfate groups. The dendritic polyol derivatives provide a unimolecular structure, wherein the interior space defines a region for the entrapment of hydrophobic drugs and the outer surface defines a negatively charged, hydrophilic region. The solubility of hydrophobic drugs can be apparently improved by protonation of one or more cyclic amines of the drug molecules and entrapment within the core region of the dendritic polymer. Thus, delivery of poorly water-soluble drugs having one or more cyclic amine functions can be obtained by a pharmaceutical formulation with hyperbranched polyester polyol derivatives and administration of a pharmaceutical composition to a mammal.

Aspects of the present invention are described in detail below. The hyperbranched polyester polyol acts as solubilizing agent (component A) for solubilizing a hydrophobic molecule with one or more cyclic amine functions (component B) in an aqueous solvent (component C). Incidentally, the term "solubilization" used in the present disclosure means preparing a thermodynamically stable solution by dispersing the hydrophobic molecule with one or more cyclic amine functions (component B) in the aqueous solvent (component C), or bringing the system into a state of homogeneous phase such as a micro-emulsion state.

The component A (solubilizing agent) functions for solubilizing the component B (hydrophobic molecule with one or more cyclic amine function) in the aqueous solvent, and comprises as an effective ingredient, a sulfated, dendritic polyester polyol having, in an embodiment, a degree of sulfation not less than 30% of available hydroxyl groups.

Table 1 lists selected examples of hydrophobic heterocyclic tumor signal transduction inhibitors comprising at least one cyclic amine suited to be solubilized by an aqueous solution containing a hyperbranched polyester polyol derivative. The increase of solubility was in the range of factor 3 to 100.

**Table 1:** Drug compounds highly suited for solubilization by an aqueous solution of the hyperbranched polyester polyol derivative.

| Compound | $pk_s$ / $pk_b$ | Solubility in water mg/ml | Solubility at pH 1.2 | Solubility in aqueous polymer solution mg/ml |
|---|---|---|---|---|
| **Sunitinib** | 11.4 / 9.0 | 0.03 | +++ | > 0.3 |
| **Trametinib** | 12.6 / -3.2 | 0.03 | - | > 0.3 |
| **Dabrafenib** | 7.2 / 2.9 | 0.03 | + | > 0.3 |
| **Bortezomib** | 13.0 / -0.7 | 0.05 | - | > 0.3 |
| **Talazoparib** | 9.5 / 1.7 | 0.10 | - | > 0.3 |

(continued)

| Compound | $pk_s$ / $pk_b$ | Solubility in water mg/ml | Solubility at pH 1.2 | Solubility in aqueous polymer solution mg/ml |
|---|---|---|---|---|
| **Osimertinib** | 13.6 / 8.8 | 0.02 | +++ | > 0.3 |
| **Gefinitib** | 16.1 / 6.8 | 0.02 | +++ | > 0.3 |
| **Afatinib** | 12.5 / 8.8 | 0.01 | +++ | > 0.3 |
| **Erlotinib** | 16.1 / 4.6 | 0.01 | + | > 0.3 |
| **Lapatinib** | 15.9 / 7.2 | 0.02 | +++ | > 0.3 |
| **Neratinib** | 12.9 / 8.8 | 0.006 | +++ | > 0.3 |
| **Dacomitinib** | 12.5 / 8.5 | 0.009 | +++ | > 0.3 |
| **Bosutinib** | 15.5 / 8.4 | 0.01 | +++ | > 0.3 |
| **Dasatinib** | 8.5 / 7.2 | 0.01 | +++ | > 0.3 |
| **Imatinib** | 12.4 / 8.2 | 0.01 | +++ | > 0.3 |
| **Nilotinib** | 12.4 / 5.9 | 0.002 | ++ | > 0.3 |
| **Ponatinib** | 11.3 / 8.0 | 0.002 | +++ | > 0.3 |
| **Ibrutinib** | 19.7 / 6.6 | 0.02 | +++ | > 0.3 |
| **Cabozantinib** | 13.5 / 5.9 | 0.002 | +++ | > 0.3 |
| **Pazopanib** | 10.4 / 4.9 | 0.04 | + | > 0.3 |
| **Regorafenib** | 10.5 / 2.0 | 0.001 | - | > 0.3 |
| **Vemurafenib** | 7.2 / 3.2 | 0.0003 | - | > 0.3 |
| **Rucaparib** | 13.2 / 9.3 | 0.01 | +++ | > 0.3 |
| **Olaparib** | 8.7 / 2.2 | 0.12 | - | > 0.3 |
| **Niraparib** | 14.0 / 10.0 | 0.01 | - | > 0.3 |
| **Selumetinib** | 6.5 / 5.4 | 0.04 | + | > 0.3 |
| **Entrectinib** | 12.4 / 7.8 | 0.009 | +++ | > 0.3 |
| **Idasanutlin** | 3.9 / 7.1 | 0.001 | n.t. | > 0.3 |
| **Ipatasertib** | 13.7 / 9.5 | 0.05 | n.t. | > 0.3 |
| **Lorlatinib** | 19.7 / 5.7 | 0.10 | +++ | > 0.3 |
| **Axitinib** | 13.1 / 4.6 | 0.0005 | ++ | > 0.3 |
| **Glasdegib** | 11.4 / 6.7 | 0.05 | +++ | > 0.3 |
| **Gedatolisib** | 11.2 / 9.7 | 0.04 | n.t. | > 0.3 |
| **Barasertib** | 1.5 / 9.5 | 0.05 | n.t. | > 0.3 |
| **Encorafenib** | 8.4 / 3.1 | 0.01 | ++ | > 0.3 |
| **Binimetinib** | 10.2 / 5.3 | 0.05 | +++ | > 0.3 |
| **Cobimetinib** | 13.4 / 9.8 | 0.04 | +++ | > 0.3 |
| **Ruxolitinib** | 13.9 / 5.5 | 0.10 | n.t. | > 0.3 |
| **SAR405838** | 12.2 / 8.8 | 0.002 | n.t. | > 0.3 |
| **MI-773** | | 0.001 | n.t. | > 0.3 |
| **APG-115** | | 0.002 | n.t. | > 0.3 |
| **Siremadlin** | | 0.001 | n.t. | > 0.3 |

(continued)

| Compound | $pk_s$ / $pk_b$ | Solubility in water mg/ml | Solubility at pH 1.2 | Solubility in aqueous polymer solution mg/ml |
|---|---|---|---|---|
| **Staurosporin** | 14.1 / 9.5 | 0.04 | n.t. | > 0.3 |
| **Capivarsetib** | 4.6 / 9.4 | 0.08 | n.t. | > 0.3 |
| **Uprosertib** | 14.0 / 9.9 | 0.03 | n.t. | > 0.3 |
| **GSK2110183** | 13.9 / 9.0 | 0.007 | n.t. | > 0.3 |
| **Miransertib** | | 0.06 | n.t. | > 0.3 |
| **BAY1125976** | | 0.001 | n.t. | > 0.3 |
| **Ravoxertinib** | | 0.004 | n.t. | > 0.3 |
| **Ulixertinib** | 12.8 / 4.6 | 0.004 | n.t. | > 0.3 |
| **Fimepinostat** | | 0.002 | n.t. | > 0.3 |
| **Mocetinostat** | 13.9 / 4.3 | 0.008 | n.t. | > 0.3 |
| **Belinostat** | 7.8 / -5.1 | 0.03 | n.t. | > 0.3 |
| **Entinostat** | 13.4 / 4.7 | 0.005 | n.t. | > 0.3 |
| **Alpelisib** | 7.8 / 2.8 | 0.005 | n.t. | > 0.3 |
| **GSK343** | | 0.001 | n.t. | > 0.3 |
| **Nedisertib** | | 0.08 | n.t. | > 0.3 |
| **LY3023414** | 14.3 / 4.3 | 0.08 | n.t. | > 0.3 |
| n.t. - not tested | | | | |

Table 2 lists, on the other hand, selected examples of hydrophobic small drug molecules which do not comprise at least one cyclic amine and therefore cannot be efficiently solubilized by an aqueous solution containing a hyperbranched polyester polyol derivative. These substances represent a negative control, confirming the necessity of the presence of at least a cyclic amine.

**Table 2:** Drug compounds not suited for solubilization by an aqueous solution of the hyperbranched polyester polyol derivative.

| Compound | $pk_s$ / $pk_b$ | Solubility in water mg/ml | Solubility in aqueous polymer solution mg/ml |
|---|---|---|---|
| **Dexamethasone** | 12.4 / -3.2 | 0.05 | < 0.1 |
| **Curcumin** | 9.1 / -4.4 | 0.006 | < 0.1 |
| **Ibuprofen** | 4.85 | 0.07 | < 0.1 |

The chemical structure of the selected hydrophobic small drug molecules which do not contain at least an amine are reported in Table 3.

**Table 3:** Chemical structure of drugs not suited for the solubilization by an aqueous solution of the hyperbranched polyester polyol derivative.

| | |
|---|---|
| Dexamethasone | 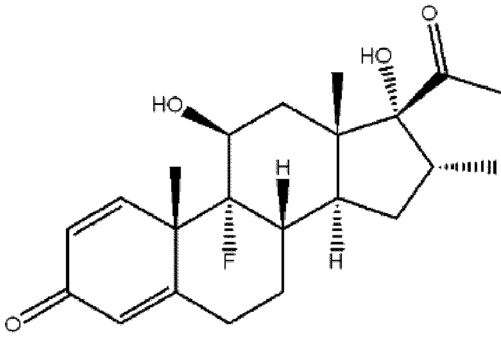 |
| Curcumin | 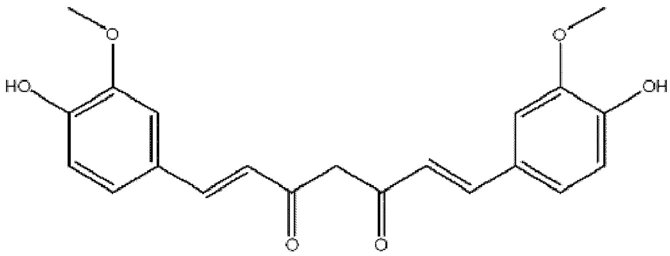 |
| Ibuprofen | 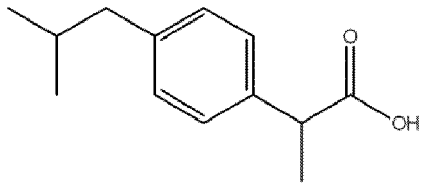 |

The component C is an aqueous solvent which consist of or comprises water and serves as a solvent for dissolving the component B therein. Component C is, in an embodiment, deionized water which has been subjected to ion exchange treatment to remove anions and cations therefrom. In an embodiment, component C is Milli-Q water, i.e., deionized water additionally filtered to remove microorganisms.

In an embodiment, the reaction for manufacturing a hyperbranched polyester polyol derivative is carried out as one-pot reaction. In an embodiment, it only consists of the above-explained steps (and optional purification steps). Thus, the hyperbranched polyester polyol derivative can be manufactured in an extremely simple manner. It cannot be used only as carrier for heterocyclic tumor signal transduction inhibitors comprising at least one cyclic amine, but has anti-inflammatory properties, i.e., it is pharmaceutically active.

Due to its simplicity, it is possible to perform this manufacturing method in technically large-scale so that it is suited to produce significant amounts of the hyperbranched polyester polyol derivative.

It is neither necessary nor intended to copolymerize any other substances than glycidol and $\varepsilon$-caprolactone. The first step of the method for manufacturing a hyperbranched polyester polyol derivative specifically does not make use of any other substances than glycidol and $\varepsilon$-caprolactone to be polymerized. To be more precise, no coupling reagents like hexamethylene diisocyanate (HDI) are used. In addition, no aromatic substances are used to be copolymerized for building up the hyperbranched polyester polyol derivative.

Due to the copolymerized caprolactone residues, the hyperbranched polyester polyol derivative comprises aliphatic hydrophobic segments. Together with hydrophilic glycerol residues, an overall amphiphilic structure results. It is particularly appropriate for encapsulating hydrophobic heterocyclic tumor signal transduction inhibitors comprising at least one cyclic amine.

The hyperbranched polyester polyol derivative has no core-shell structure, but rather shows a random structure having a statistical distribution of hydrophobic caprolactone residues within hydrophilic glycerol residues. A significant part of hydroxyl groups of the glycerol residues is sulfated.

In an embodiment, the first step of the method for manufacturing a hyperbranched polyester polyol derivative is carried out in the presence of a catalyst. It turned out that a Lewis acid such as a tin compound acting as Lewis acid is a particularly appropriate catalyst for this reaction step. In an embodiment, tin(II) 2-ethylhexanoate (tin octoate, $Sn(Oct)_2$) is used as catalyst. Experimental data revealed that other catalysts such as Novozym 435 are likewise appropriate. Furthermore, the reaction can be carried out without any catalysts as further experiments of the inventors revealed. Then, only an elevated temperature lying in a range of 30 °C and 150 °C, in particular 30 °C to 70 °C or any of the temperature ranges mentioned in the next paragraph appears favorable to achieve a sufficiently high yield.

The reaction can be carried out under mild (ambient) conditions. In an embodiment, the reaction is carried out at a reaction temperature lying in a range of between 30 °C and 150 °C, in particular between 40 °C and 140 °C, in particular between 50 °C and 130 °C, in particular between 60 °C and 120 °C, in particular between 70 °C and 110 °C, in particular between 80 °C and 105 °C, in particular between 90 °C and 100 °C. Particular appropriate reaction temperatures are temperatures at or around 50 °C, at or around 90 °C, and at or around 120 °C.

In an embodiment, a molar ratio between glycidol and ε-caprolactone is between 1 to 1 and 10 to 1, in particular between 2 to 1 and 9 to 1, in particular between 3 to 1 and 8 to 1, in particular between 4 to 1 and 7 to 1, in particular between 5 to 1 and 6 to 1. Thus, it is always used at least as much glycidol as ε-caprolactone, but particularly more glycidol than ε-caprolactone. Particular appropriate molar ratios between glycidol and ε-caprolactone are molar ratios of 2 to 1 and 4 to 1.

Obviously, the molar ratio between glycidol and ε-caprolactone influences the structure of the resulting hyperbranched polyester polyol. In addition, the reaction temperature also influences the resulting structure. Particular appropriate combinations between molar ratios and reaction temperatures are a molar ratio of 2 to 1 and a temperature of 50 °C, 90 °C, or 120 °C as well as a molar ratio of 4 to 1 and a temperature of 50 °C, 90 °C, or 120 °C.

The sulfation reagent used in the second step of the method is, in an embodiment, a sulfur trioxide base complex (e.g., $SO_3$·Pyridine, or $SO_3$·triethylamine). Sulfur trioxide pyridine complex is particularly appropriate. The sulfation can be carried out as generally known in the art. It takes place at the free hydroxyl groups of the hyperbranched polyester polyol so that a sulfated hyperbranched polyester polyol results.

Appropriate reaction conditions for the sulfation step comprise a reaction temperature of 40 °C to 80 °C, in particular of 45 °C to 75 °C, in particular of 50 °C to 70 °C, in particular of 55 °C to 65 °C, in particular of 60 °C to 80 °C, and/or a reaction duration of 12 hours to 2 days, in particular of 1 day to 1.5 days. Particular appropriate reaction conditions are a reaction temperature of 55 °C to 65 °C (such as 60 °C) and a reaction duration of approximately one day.

As already stated above, the concrete structure of a hyperbranched polyester polyol derivative that can be obtained by a method according to the preceding explanations cannot be exactly described in more concrete terms than by making reference to its manufacturing method since the reaction temperature and the molar ratio of the educts, i.e., of glycidol and of ε-caprolactone, influence the resulting molecular structure of the hyperbranched polyester polyol derivative.

In an embodiment, the hyperbranched polyester polyol derivative has a degree of sulfation of from 30 % to 100 %, in particular of from 40 % to 95 %, in particular of from 50 % to 90 %, in particular of from 60 % to 80 %, in particular of from 70 to 100 %, in particular of from 75 to 99 %, in particular of from 80 to 98 %, in particular of from 85 to 97 %, in particular of from 90 to 95 %. Thus, a significant amount of hydroxyl groups of the hyperbranched polyester polyol resulting from the first step of the method is sulfated and the second step of the manufacturing method according to this embodiment.

"Degree of sulfation" according to this disclosure means the percentage of functionalized OH groups of the glycerol units of the hyperbranched polyester polyol. The functionalization results from the substitution of one or more OH groups of the glycerin units with $-OSO_3H$ or $-OSO_3^-K^+$ (K being, e.g., Na) groups.

In an embodiment, the hyperbranched polyester polyol derivative has a number average molecular weight lying in a range of between 25 and 75 kDa, in particular of between 30 and 70 kDa, in particular of between 35 and 65 kDa, in particular of between 40 and 60 kDa, in particular of between 45 and 55 kDa.

In an embodiment, the hyperbranched polyester polyol derivative has a branching degree of 30 % to 100 %, in particular 40 % to 95 %, in particular 50 % to 90 %, in particular 60 % to 85 %, in particular 70 % to 80 %.

Regarding further details, aspect and embodiments of the manufacturing method for the hyperbranched polyester polyol derivative to be used in a composition is claimed to herein, reference is made to WO 2019/096782 A1. The details, aspect and embodiments explained in that international patent application can be fully applied within aspects of the presently claimed invention.

In an embodiment, the heterocyclic tumor signal transduction inhibitor is at least one chosen from the group consisting of sunitinib, trametinib, dabrafenib, topotecan, idasanutlin, ruxolitinib, borussertib, talazoparib, and S 2046. These compounds have the chemical structures shown in Table 4.

**Table 4:** Chemical structures of selected heterocyclic tumor signal transduction inhibitors.

| | |
|---|---|
| Sunitinib | 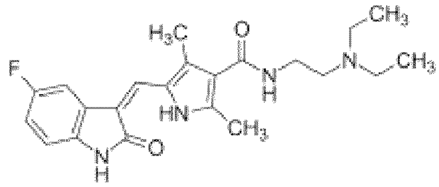 |
| Trametinib | 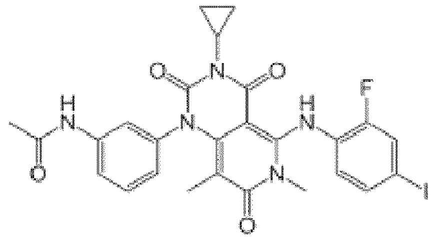 |
| Dabrafenib | 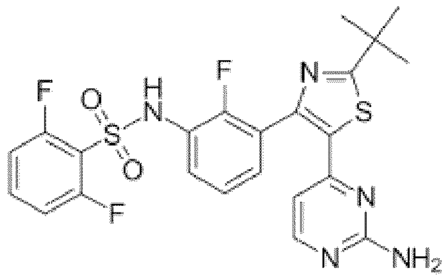 |
| Topotecan | 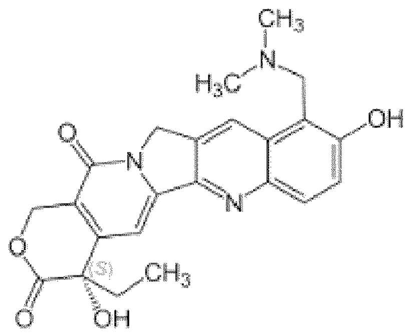 |
| Idasanutlin | 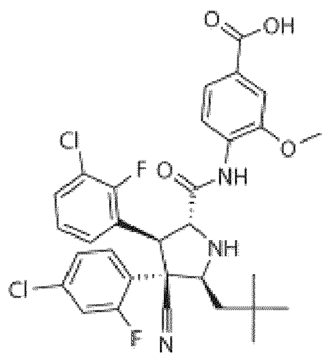 |

(continued)

| | |
|---|---|
| Ruxolitinib | 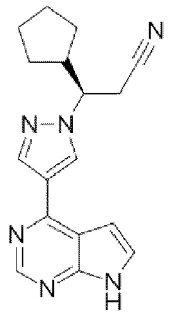 |
| Borussertib | 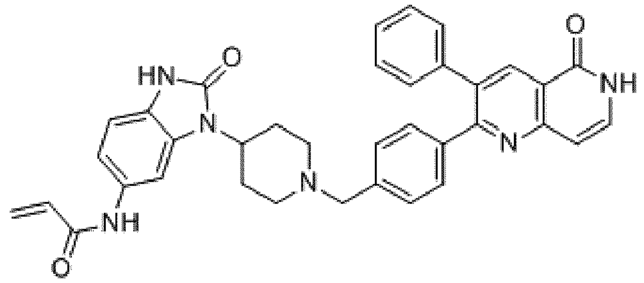 |
| Talazoparib | 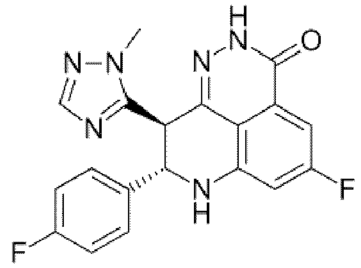 |

In an embodiment, the heterocyclic tumor signal transduction inhibitor is encapsulated by the hyperbranched polyester polyol derivative without forming a covalent bond to the hyperbranched polyester polyol derivative. In such a case, hydrophobic interactions, van-der-Waals interactions or other non-covalent interactions can be established between the heterocyclic tumor signal transduction inhibitor and the hyperbranched polyester polyol derivative to stabilize the inhibitor by the hyperbranched polyester polyol derivative.

In an embodiment, the composition comprises an aqueous solution in which the hyperbranched polyester polyol derivative and the heterocyclic tumor signal transduction inhibitor are dissolved or dispersed. In an embodiment, the composition does not comprise any organic solvent. Thus, it is possible to work fully without any organic solvents when applying the presently described and claimed composition. This facilitates the manufacturing of the composition and also facilitates its administration to a human or animal patient since no potentially harmful organic solvents need to be removed prior to administering the composition.

In an aspect, the present invention relates to the medical use of a composition as described above. In this context, the composition can be used as therapeutic or diagnostic medicament.

In an aspect, the present invention relates to the further medical use of a composition as described above for treating a tumor or treating cancer in a patient in need thereof.

In an aspect, the present invention relates to a method for manufacturing a composition according to the preceding explanations. This manufacturing method comprises the steps explained the following.

In a first step, a heterocyclic tumor signal transduction inhibitor is provided in dry form or in form of an aqueous solution. The heterocyclic tumor signal transduction inhibitor comprises at least one cyclic amine.

In a second step, an aqueous solution of the hyperbranched polyester polyol derivative is added to the heterocyclic tumor signal transduction inhibitor.

Afterwards, the mixture obtained in the preceding method step is agitated to obtain a composition of heterocyclic tumor signal transduction inhibitor encapsulated by the hyperbranched polyester polyol derivative. The agitation can be performed, e.g., by stirring, shaking or by otherwise introducing kinetic energy into the mixture.

Since typically not the whole amount of heterocyclic tumor signal transduction inhibitor is in fact encapsulated

by the hyperbranched polyester polyol derivative, it is appropriate to separate the non-encapsulated heterocyclic tumor signal transduction inhibitor from the composition comprising the heterocyclic tumor signal transduction inhibitor encapsulated by the hyperbranched polyester polyol derivative. This purification step serves for providing a composition comprising heterocyclic tumor signal transduction inhibitor encapsulated by the hyperbranched polyester polyol derivative but being essentially devoid of any free (non-encapsulated) heterocyclic tumor signal transduction inhibitor.

In an embodiment, the separating step is performed by subjecting the mixture of the heterocyclic tumor signal transduction inhibitor and the hyperbranched polyester polyol derivative after the agitation step to an appropriate separation or purification method. Centrifugation and/or a chromatography are particularly appropriate separation or purification techniques. By centrifugation, the non-encapsulated heterocyclic signal transduction inhibitor having a lower molecular mass than the complex of hyperbranched polyester polyol derivative with encapsulated heterocyclic tumor signal transduction inhibitor can be typically well separated from this complex. Appropriate chromatography techniques are gas chromatography, liquid chromatography, affinity chromatography, supercritical fluid chromatography, ion exchange chromatography, size-exclusion chromatography, expanded bed adsorption chromatographic separation, reversed-phase chromatography, hydrophobic interaction chromatography, two-dimensional chromatography, simulated moving-bed chromatography, counter current chromatography, periodic counter-current chromatography, and aqueous normal-phase chromatography. Size-exclusion chromatography (sometimes also referred to as gel permeation chromatography or gel filtration chromatography) is a particularly appropriate technique to separate molecules having a different size or molecular weight from each other.

In an embodiment, a centrifugation is combined with a size-exclusion chromatography to purify the obtained heterocyclic tumor signal transduction inhibitor encapsulated by the hyperbranched polyester polyol derivative, in particular to separate it from non-encapsulated heterocyclic tumor signal transduction inhibitor.

Examples of the improved solubilization of the drug by the hyperbranched polyester polyol derivative are reported in Table 5 and 6 with respect to exemplary embodiments.

In an embodiment, the composition of heterocyclic tumor signal transduction inhibitor encapsulated by the hyperbranched polyester polyol derivative is freeze-dried after the separating/purification step. As reported in Table 7 with respect to an exemplary embodiment, the process is not resulting in change of the size of the hyperbranched polyester polyol derivative and is only partially affecting its surface charge. It also turned out that the biological activity of the encapsulated heterocyclic tumor signal transduction inhibitor is not impaired by such freeze-drying process, as reported in Figure 1, Figure 2 and Table 8 with respect to exemplary embodiments. At the same time, the shelf life of freeze-dried composition is much longer than the shelf life of the composition in aqueous solution. Hydrophobic compounds in other preparations can often not be freeze-dried since they destabilize or otherwise disintegrate during such a lyophilization process. In contrast, the heterocyclic tumor signal transduction inhibitor stabilized by the hyperbranched polyester polyol derivative does not show such adverse behavior but rather enables much easier storage and transportation possibilities than formulations of hydrophobic drugs used in prior art.

In an embodiment, the manufacturing method is carried out only in aqueous solutions, i.e., without using organic solvents. Typically, hydrophobic heterocyclic tumor signal transduction inhibitors or other hydrophobic drugs require solubilization in an organic solvent to be processed. The presently described novel possibility of stabilizing and solubilizing hydrophobic heterocyclic tumor signal transduction inhibitors by the hyperbranched polyester polyol derivative enables working without use of any organic solvents. This enhances medicament safety and patient safety since no traces of incompletely removed organic solvent can be present in a medicament produced from the composition as described herein.

For manufacturing a composition according to aspects of the present invention, a predetermined amount of the solubilizing agent (component A) and the aqueous solvent (component C) is weighted, mixed and agitated together until a clear aqueous solution is achieved. Then, a predetermined amount of the hydrophobic molecule (component B) is added to the aqueous solution of components A and C. Solubilizing of the hydrophobic molecule (component B) by an aqueous solution of component A and C is achieved by stirring, mixing or shaking. It is not necessary to apply intense mechanical forces to achieve solubilization of component B. However, as a matter of course, it is not excluded to conduct such an intense mechanical agitation capable of applying a strong shear force to the composition.

In an aspect, the invention relates to a medical method comprising administering a composition according to the preceding explanations to a person in need thereof. This medical method is a method for treatment and/or diagnosis of the person.

The composition as explained herein can be provided, for example, when used as medicament, in form of pharmaceutical compositions, comprising additionally a pharmaceutical acceptable carrier. Preferably, such a pharmaceutical composition has a unit dosage form, such as tablets, pills, capsules, powder, granulate, sterile parenteral solutions or suspensions. Further dosage forms are known to the person of skill in the art and may be applied.

A medicament or a pharmaceutical composition comprises a therapeutically effective amount of the composition as explained herein. A skilled person will be able to determine the therapeutically effective amount on the basis of the disease to be treated and in consideration of the state of the patient. A medicament or a pharmaceutical composition

can suitably contain between about 5 mg and 1000 mg, in particular between about 10 mg and 900 mg, in particular between about 25 and 800 mg, in particular between about 50 mg and 700 mg, in particular between about 100 mg and 600 mg, in particular between about 200 mg and 500 mg, in particular between about 300 mg and 400 mg of a composition according to the present explanations.

Summarizing, the aspects of the presently claimed and described invention relate to the provision of a solubilizing agent exhibiting a higher solubilizing property for solubilizing hydrophobic, poorly-water soluble molecules with one or more cyclic amine functions in an aqueous solvent, and of a hydrosol composition containing the solubilized hydrophobic molecules, which composition can be readily prepared, kept stable for a long period of time and used for treatment and diagnosis.

All variants and embodiments of the described composition can be combined in any desired way and can be transferred individually or in any combination to the described uses and the described manufacturing method. Likewise, all variants and embodiments of the described uses can be combined in any desired way and can be transferred individually or in any desired combination to the described composition and the described manufacturing method. Furthermore, all variants and embodiments of the described manufacturing method can be combined in any desired way and can be transferred to the described composition and the uses of this composition.

Further details of aspects of the present invention will be explained with respect to exemplary embodiments and accompanying Figures. In the Figures:

Figure 1 shows the toxicity of sunitinib toward HeLa cells when comparing free sunitinib to sunitinib solubilized by the hyperbranched polyester polyol derivative as carrier; and

Figure 2 shows the toxicity of sunitinib toward KB-V1 cells when comparing free sunitinib to sunitinib solubilized by the hyperbranched polyester polyol derivative as carrier.

## STANDARD FORMULATION PROCESS

The encapsulation of the heterocyclic tumor signal transduction inhibitor comprising at least one cyclic amine (drug) is conducted in aqueous medium, at room temperature. To the dry drug, in powder, an aqueous solution of a hyperbranched polyester polyol derivative (polymer) manufactured according to the procedure explained in WO 2019/096782 A1, freshly dissolved, is added. Typical setting: 10 mg drug, 100 mg polymer dissolved in 10 mL Milli-Q water. The solution is stirred at 1000 rpm, in dark, for 18 h. Afterwards the solution is centrifuged at 3000 rpm for 5 minutes. For colored drugs, the solution is additionally passed on a sephadex column. The solution is then lyophilized and stored at -20 °C.

## SEPHADEX PURIFICATION

For the separation of non-encapsulated drug from the solubilized (encapsulated) drug, the solution is passed on a sephadex column (size-exclusion chromatography), where the particles are separated on the base of the molecular weight. Sephadex: G25 (healthcare). Column length: ca. 20 cm. Depending on the volume and amount of formulation, the length can be adjusted (either reduced or expanded). Prior to utilization, the sephadex powder is swelled in water for at least 2 h. Generally, the sephadex is conserved in water to be ready to use.

## MANUFACTURING OF WATERLESS FORMULATION

The formulation is dissolved in a small volume of water, freeze-dried and hanged on the lyophilization instrument to remove all the water for at least 24 h.

## EXAMPLE #1: SUNITINIB

The encapsulation is conducted according to the standard formulation process. After centrifuging the solution at 3000 rpm for 5 minutes, the supernatant is additionally passed on a sephadex column, G25. Column length: ca. 20 cm. The solution is then lyophilized and stored at -20 °C.

## EXAMPLE #2: DABRAFENIB

The encapsulation is conducted as in case of sunitinib.

## EXAMPLE #3: TRAMETINIB

The encapsulation is conducted as in case of sunitinib.

## pH CONTROL FORMULATION PROCESS

The encapsulation is conducted in aqueous medium, in acidic pH, at room temperature. HCl is added to the Milli-Q water to get a pH of 2. This solution is then used to dissolve the polymer and added to the dry drug. Typical setting: 10 mg drug, 100 mg polymer dissolved in 10 mL Milli-Q water. The solution is stirred at 1000 rpm, in dark, for 18 h. Afterwards the solution is centrifuged at 3000 rpm for 5 minutes. For colored drugs, the solution is additionally passed on a sephadex column. The solution is then lyophilized and stored at -20 °C.

## EXAMPLE #4: BORUSSERTIB

The encapsulation is conducted according to the pH control formulation process. After centrifuging the solution at 3000 rpm for 5 minutes, the supernatant is recovered, lyophilized and stored dry at -20 °C.

## QUANTIFICATION OF THE DRUG LOADING BY UV-VIS

For drugs presenting an absorption band at wavelength higher than 400 nm, UV-VIS analysis is chosen as quantification method. First, a calibration curve of the free drug in the suitable solvent mixture is recorded. Suitable combinations of solvent include mixtures of water and methanol, as well as of water and ethanol, in both cases in a ratio falling in a range of from 40 to 60 % to 20 to 80 %.

The calibration curve is prepared with at least 5 points, in a concentration range depending on the drug to be quantified. Linear regression is applied to generate the equation of the straight-line necessary to calculate the drug content.

The formulation is afterwards dissolved in the same medium and an UV-VIS spectrum is recorded. It is possible to use a straight-line equation to get the concentration of drug in solution. Knowing the total volume of the formulation, the content of drug, in mg, can be calculated.

The carrying potential (CP) of the polymer (solubilizing agent) is obtained as the ratio of the loaded and solubilized drug to the amount of solubilizing agent (Eq. 1).

$$CP = \frac{drug\ (mg)}{carrier\ amount\ (mg)} * 100$$

## QUANTIFICATION OF THE DRUG LOADING BY ELEMENTAL ANALYSIS

For drugs presenting an absorption band at wavelength lower than 400 nm, the UV-VIS approach is not suitable, due to drug signals overlapping with signals of the solubilizing agent. Therefore, elemental analysis is chosen as alternative approach. The drugs chosen for the solubilization and entrapment contain nitrogen atoms, which are not present in the polymer structure. Via the quantification of the nitrogen content in the sample, it is possible to determine the amount of drug in it. For the loading quantification, the same equation is used as in the previous case.

The following tables (Table 5, Table 6 and Table 7) show the results of the performed analyses. In this context, $h(PG\text{-}co\text{-}PCL)_{60}S_{0.95}$ was used as hyperbranched polyester polyol derivative. Its non-sulfated backbone has a molecular weight of 60 kDa. The polyester polyol derivative has a sulfation degree of 95 %.

**Table 5:** Carrying potential of $h(PG\text{-}co\text{-}PCL)_{60}S_{0.95}$ calculated by elemental analysis

| Drug | Polymer amount (mg) | Drug amount (mg) | Carrying potential (wt%) |
|---|---|---|---|
| **Sunitinib** | 1.14 | 0.099 | 8.7% |
| **Dabrafenib** | 508.71 | 38.29 | 7.5% |
| **Trametinib** | 58.8 | 11.2 | 16.5% |
| **Borussertib** | 1.62 | 0.197 | 12% |

**Table 6:** Carrying potential of h(PG-co-PCL)$_{60}$S$_{0.95}$ calculated by UV

| Drug | Polymer amount (mg) | Drug amount (mg) | Carrying potential (wt%) |
|---|---|---|---|
| **Sunitinib** | 236 | 25.54 | 11 % |

**Table 7:** Size and Z-potential investigation of h(PG-co-PCL)$_{60}$S$_{0.95}$ - DLS in PB

| Solubilized Drug | Size (volume) | Z-potential |
|---|---|---|
| **Nude Polymer** | 12 ± 4 | -48.9 |
| **Sunitinib** | 14 ± 6 | -33.5 |
| **Dabrafenib** | 12 ± 1 | -29.6 |
| **Trametinib** | 14 ± 2 | -18.9 |

Figure 1 shows the results of a first cell viability experiments in which the toxicity of free sunitinib was compared to that of sunitinib (heterocyclic tumor signal transduction inhibitor comprising at least one cyclic amine) encapsulated by h(PG-co-PCL)$_{60}$S$_{0.95}$ (hyperbranched polyester polyol derivative). From these experiments, the IC$_{50}$ values of free and encapsulated sunitinib for HeLa cells can be derived.

Figure 2 shows the results of a second cell viability experiments in which the toxicity of free sunitinib was compared to that of sunitinib encapsulated by h(PG-co-PCL)$_{60}$S$_{0.95}$. From these experiments, the IC$_{50}$ values of free and encapsulated sunitinib for KB-V1 cells can be derived.

**Table 8:** IC$_{50}$ values comparison of the drugs solubilized in DMSO and drugs solubilized in sulfated polyester polyols

| Compound | Cell line | IC$_{50}$ (μg/mL) |
|---|---|---|
| **Sunitinib free drug** | Hela | 3.523 |
| **h(PG-co-PCL)$_{60}$S$_{0.95}$ @ sun** | Hela | 5.685 |
| **Sunitinib free drug** | KB-V1 | 1.954 |
| **h(PG-co-PCL)$_{60}$S$_{0.95}$ @ sun** | KB-V1 | 2.951 |

## Claims

1. Composition comprising

   i) a hyperbranched polyester polyol derivative obtainable by a method comprising the following steps:

      a) reacting only glycidol and ε-caprolactone at a temperature lying in a range of between 40 °C and 140 °C to obtain a hyperbranched polyester polyol derivative in which caprolactone residues and glycerol residues are randomly arranged,
      b) reacting the hyperbranched polyester polyol derivative of step a) with a sulfation reagent to obtain a sulfated hyperbranched polyester polyol as hyperbranched polyester polyol derivative

   ii) and a heterocyclic tumor signal transduction inhibitor comprising at least one cyclic amine.

2. Composition according to claim 1, **characterized in that** the heterocyclic tumor signal transduction inhibitor is at least one chosen from the group consisting of sunitinib, trametinib, dabrafenib, bortezomib, talazoparib, osimertinib, gefinitib, afatinib, erlotinib, lapatinib, neratinib, dacomitinib, bosutinib, dasatinib, imatinib, nilotinib, ponatinib, ibrutinib, cabozantinib, pazopanib, regorafenib, vemurafenib, rucaparib, olaparib, niraparib, selumetinib, entrectinib, idasanutlin, ipatasertib, lorlatinib, axitinib, glasdegib, gedatolisib, barasertib, encorafenib, binimetinib, cobimetinib, ruxolitinib, SAR405838, MI-773, APG-115, siremadlin, staurosporin, capivarsetib, uprosertib, GSK2110183, miransertib, BAY1125976, ravoxertinib, ulixertinib, fimepinostat, mocetinostat, belinostat, entinostat, alpelisib, GSK343, nedisertib, and LY3023414.

3. Composition according to claim 1 or 2, **characterized in that** the heterocyclic tumor signal transduction inhibitor is at least one chosen from the group consisting of sunitinib, trametinib, dabrafenib, topotecan, idasanutlin, ruxolitinib, borussertib, and talazoparib.

4. Composition according to any of the preceding claims, **characterized in that** no covalent bond is formed between the hyperbranched polyester polyol derivative and the heterocyclic tumor signal transduction inhibitor.

5. Composition according to any of the preceding claims, **characterized in that** the composition comprises an aqueous solvent.

6. Composition according to any of the preceding claims for use as a medicament.

7. Composition according to any of claims 1 to 5 for use in treating a tumor.

8. Method for manufacturing a composition according to any of claims 1 to 5, comprising the following steps:

   a) providing a heterocyclic tumor signal transduction inhibitor in dry form or in form of an aqueous solution, wherein the heterocyclic tumor signal transduction inhibitor comprises at least one cyclic amine,
   b) adding an aqueous solution of the hyperbranched polyester polyol derivative to the heterocyclic tumor signal transduction inhibitor,
   c) agitating the mixture obtained in the preceding step to obtain a composition of heterocyclic tumor signal transduction inhibitor encapsulated by the hyperbranched polyester polyol derivative, and
   d) separating non-encapsulated heterocyclic tumor signal transduction inhibitor from the composition of heterocyclic tumor signal transduction inhibitor encapsulated by the hyperbranched polyester polyol derivative.

9. Method according to claim 8, **characterized in that** the separating step is performed by subjecting the mixture after step c) to at least one of a centrifugation and a chromatography.

10. Method according to claim 8 or 9, **characterized in that** the composition of heterocyclic tumor signal transduction inhibitor encapsulated by the hyperbranched polyester polyol derivative is lyophilized after step d).

11. Method according to any of claims 8 to 10, **characterized in that** the method is carried out in no solutions other than aqueous solutions.

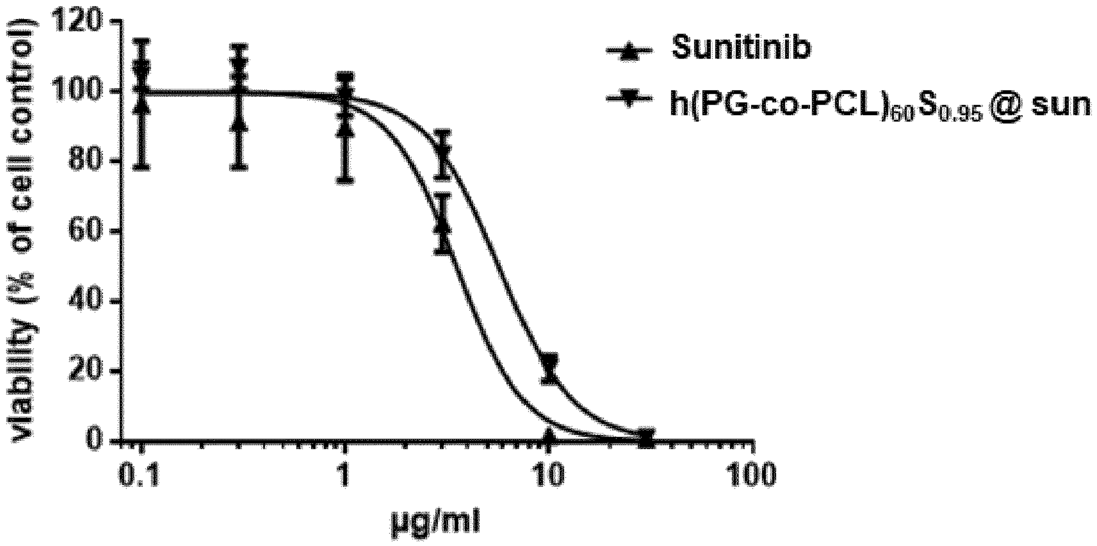
Sunitinib
h(PG-co-PCL)60S0.95@ sun
viability (% of cell control)
120
100
80
60
40
20
0
0.1
1
10
100
µg/ml

FIG 1

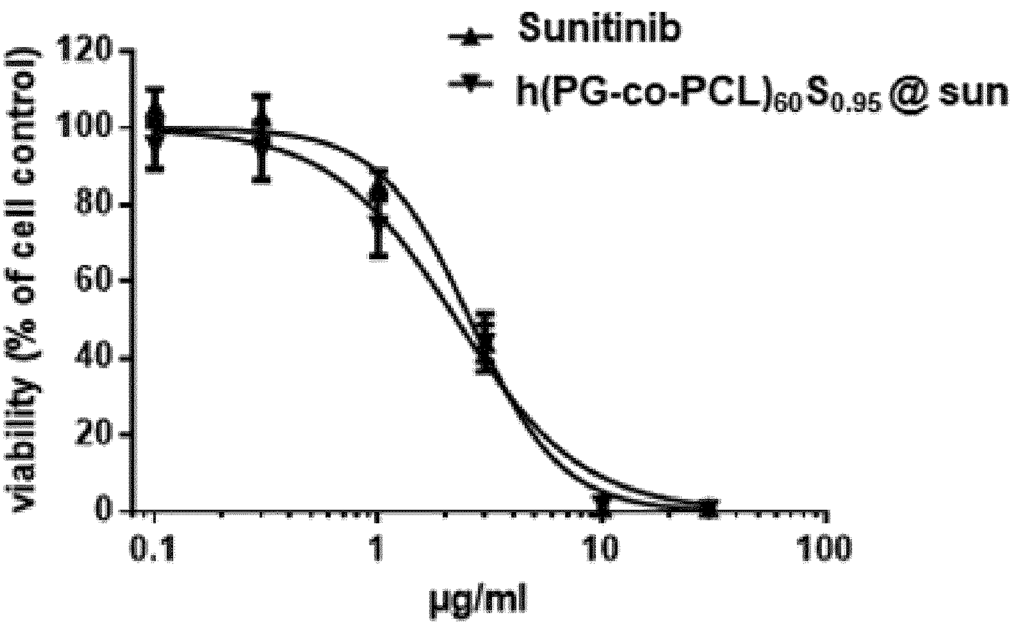
Sunitinib
h(PG-co-PCL)60S0.95@ sun
viability (% of cell control)
120
100
80
60
40
20
0
0.1
1
10
100
µg/ml

FIG 2

Europäisches Patentamt
European Patent Office
Office européen des brevets

# EUROPEAN SEARCH REPORT

Application Number
EP 20 15 4946

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CN 109 394 692 A (UNIV CHINA PHARMA) 1 March 2019 (2019-03-01) * the whole document * ----- | 1-11 | INV. A61K9/107 A61K9/19 A61K47/34 |
| A,D | WO 2019/096782 A1 (FREIE UNIV BERLIN [DE]) 23 May 2019 (2019-05-23) * the whole document * ----- | 1-11 | |
| A | MELANNIE CARNA: "Glycerol-modified poly-caprolactone nanoparticles for drug delivery application", KIMIKA, vol. 25, no. 1, 31 January 2014 (2014-01-31), page 3838, XP055453980, * the whole document * ----- | 1-11 | |

TECHNICAL FIELDS SEARCHED (IPC)

A61K

The present search report has been drawn up for all claims

1

| Place of search | Date of completion of the search | Examiner |
|---|---|---|
| Munich | 20 April 2020 | Schüle, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 20 15 4946

This annex lists the patent family members relating to the patent documents cited in the above-mentioned European search report.
The members are as contained in the European Patent Office EDP file on
The European Patent Office is in no way liable for these particulars which are merely given for the purpose of information.

20-04-2020

| Patent document cited in search report | Publication date | Patent family member(s) | Publication date |
|---|---|---|---|
| CN 109394692 A | 01-03-2019 | NONE | |
| WO 2019096782 A1 | 23-05-2019 | EP 3483201 A1 | 15-05-2019 |
| | | WO 2019096782 A1 | 23-05-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2019096782 A1